# EUROPEAN PATENT APPLICATION

(11) **EP 3 072 549 A1**
(43) Date of publication of application: **28.09.2016**
(21) Application number: 16157125.2
(22) Date of filing: 24.02.2016
(51) Int. Cl.: A61M 25/00, A61M 25/09

(54) **CATHETER**

(30) Priority: 23.03.2015 JP 2015059998; 23.03.2015 JP 2015059997; 11.12.2015 JP 2015242279
(71) Applicant: ASAHI INTECC CO., LTD., Nagoya-shi, Aichi 463-0024 (JP)
(72) Inventor: KANAZAWA, Yuya, Nagoya-shi, Aichi 463-0024 (JP); USIHDA, Keisuke, Nagoya-shi, Aichi 463-0024 (JP)
(74) Representative: Winter, Brandl, Fürniss, Hübner, Röss, Kaiser, Polte - Partnerschaft mbB

(57) **Abstract**

To provide a catheter with excellent torque transmissivity and excellent flexibility, enabling easy insertion into a blocked portion of a blood vessel or the like, and allowing the catheter to easily restore its original form even when a degree of curvature of the catheter becomes large and the catheter is deformed, thereby preventing a break at a border area between a catheter main body and a distal end tip and improving the operability of a combination device such as a guide wire. A catheter (1) includes a coil body (20) formed of at least one stranded wire (30) which is formed of a first wire (31a) and a plurality of second wires (31b) and is wound into a hollow helical coil structure.

## Description

### Field

The present invention relates to a catheter for medical use.

### Background

Conventionally, there is known a catheter including a catheter main body formed by winding an wire into a helical coil structure and a distal end tip fixed to a distal end of the catheter main body.

For example, Patent Literature 1 discloses a medical treatment instrument (catheter) in which a metal distal end tip is attached to a distal end of a coil body formed by winding metal wires.

### Citation List

### Patent Literature

Patent Literature 1: Japanese Patent Application Laid-open No. 2007-98120

### Summary

### Technical Problem

However, in the catheter described in Patent Literature 1, the coil body is formed by winding a single piece of wire into a single line (single line coil body) or a plurality of wires into a multi-line (multi-line coil body). Thus, in a single line coil body, torque transmissivity is reduced but flexibility is good. By contrast, in a multi-line coil body, flexibility is reduced but torque transmissivity is good. Therefore, it has been difficult to easily insert a catheter into a blocked portion of a blood vessel, for example.

Moreover, in the catheter described in Patent Literature 1, the catheter itself may be deformed slightly when the degree of curvature thereof becomes large. Once the catheter is deformed, its original form cannot be restored. Thus, in such a case, the operation of a combination device such as a guide wire may become difficult.

Moreover, in the catheter described in Patent Literature 1, the metal distal end tip is separately connected to the distal end of the catheter main body. Thus, stress is easily concentrated at a border part between the catheter main body and the distal end tip, easily causing a break at such a border part.

Moreover, in the catheter described in Patent Literature 1, the metal distal end tip is separately connected to the distal end of the catheter main body. Thus, a combination device such as a guide wire, which is inserted into a lumen of the catheter, may be caught at the border part between the catheter main body and the distal end tip, increasing resistance when the combination device is pushed or pulled.

Furthermore, in the catheter described in Patent Literature 1, the coil body is formed by winding a single piece of wire into a single line (single line coil body) or a plurality of wires into a multi-line (multi-line coil body), and the wire forming the coil body is inclined relative to a long axis direction of the catheter. This increases insertion resistance when the catheter is inserted into a blocked portion of a blood vessel, for example.

A first object of the present invention is to provide a catheter with excellent torque transmissivity and excellent flexibility, allowing easy insertion into a blocked portion of a blood vessel or the like.

A second object of the present invention is to provide a catheter capable of easily restoring its original form even when the degree of curvature of the catheter becomes large and the catheter is deformed.

Furthermore, a third object of the present invention is to provide a catheter capable of preventing a break at a border part between a catheter main body and a distal end tip and improving the operability of a combination device such as a guide wire.

### Solution to Problem

A first aspect of the present invention is a catheter including a coil body formed of at least one stranded wire which is formed of a plurality of single wires and is wound into a hollow helical coil structure.

A second aspect of the present invention is the catheter according to the first aspect of the invention, in which the coil body is formed of a plurality of stranded wires which are wound into a hollow helical coil structure, wherein each of the plurality of stranded wires is formed of a plurality of single wires.

A third aspect of the present invention is the catheter according to the first aspect or the second aspect of the invention, in which a distal end portion of the coil body is formed by fusing the single wires of the at least one stranded wire. In case the coil body is formed of a plurality of stranded wires, a distal end portion of the coil body may be formed by fusing all stranded wires, i.e., by fusing the single wires of all stranded wires.

A fourth aspect of the present invention is the catheter according to the first aspect or the second aspect of the invention, in which a distal end portion of the coil body includes an outer surface formed by fusing the single wires of the at least one stranded wire, and an inner surface keeping a form of the stranded wire.

A fifth aspect of the present invention is the catheter of any one of the first aspect to the fourth aspect according to the invention, further including an inner layer formed inside of the coil body and an outer layer formed outside of the inner layer so as to cover the coil body.

A sixth aspect of the present invention is a catheter according to the third aspect or the fourth aspect of the invention, further including an inner layer formed inside of the coil body, and an outer layer formed outside of the inner layer so as to cover the coil body in an area other than the distal end portion of the coil body.

### Advantageous Effects of Invention

The catheter according to the first aspect of the present invention includes a coil body formed of at least one stranded wire which is formed of a plurality of single wires and is wound into a hollow helical coil structure. In other words, the coil body may be formed, into a hollow helical coil structure, of a single (i.e., one piece of) stranded wire or of a plurality of (i.e., more than one pieces of) stranded wires. Further, the single stranded wire or each of the plurality of stranded wires is formed of a plurality of single wires which are stranded, i.e., which are twisted together. Accordingly, each stranded wire may be considered as a bundle of a plurality of (e.g., seven) single wires. Thus, the catheter has excellent torque transmissivity and excellent flexibility, improving insertion into a blocked portion of a blood vessel. Moreover, when the catheter is curved, it is possible to allow the catheter to easily restore its original form.

The catheter according to the second aspect of the present invention is the catheter according to the first aspect, in which the coil body is formed of a of stranded wires into a hollow helical coil structure. Each of the plurality of stranded wires is formed of a plurality of single wires. Thus, it is possible to further improve insertion into a blocked portion of a blood vessel and improve the operability of a combination device such as a guide wire, in addition to the effect of the catheter according to the first aspect. Moreover, when the catheter is curved, it is possible to allow the catheter to easily restore its original form more easily.

The catheter according to the third embodiment of the present invention is the catheter according to the first aspect or the catheter according to the second aspect, in which a distal end portion of the coil body is formed by fusing the single wires of the at least one stranded wire. Thus, it is possible to prevent a break at a border between a catheter main body and the distal end portion and improve the operability of a combination device such as a guide wire, in addition to the effect of the catheter according to the first aspect or the catheter according to the second aspect.

The catheter according to the fourth aspect of the present invention is the catheter according to the first aspect or the catheter according to the second aspect, in which a distal end portion of the coil body includes an outer surface formed by fusing the single wires of the at least one stranded wire, and an inner surface keeping a form of the stranded wire. Thus, it is possible to prevent a break at a border between the catheter main body and the distal end portion, improve the operability of a combination device such as a guide wire, and further improve insertion into a blocked portion of a blood vessel or the like, in addition to the effect of the catheter according to the first aspect or the catheter according to the second aspect.

The catheter according to the fifth aspect of the present invention is the catheter according to any one of the first aspect to the fourth aspect, further including an inner layer formed inside of the coil body and an outer layer formed outside of the inner layer so as to cover the coil body. Thus, it is possible to further improve insertion into a blocked portion of a blood vessel and further improve the operability of a combination device such as a guide wire, in addition to the effect of the catheter of any one of the first aspect to the fourth aspect.

The catheter according to the sixth aspect of the present invention is the catheter according to the third aspect or the fourth aspect, further including an inner layer formed inside of the coil body and an outer layer formed outside of the inner layer so as to cover the coil body in an area other than the distal end portion of the coil body. Thus, it is possible to further improve insertion into a blocked portion of a blood vessel and further improve the operability of a combination device such as a guide wire, in addition to the effect of the catheter according to the third aspect or the catheter according to the fourth aspect.

### Brief Description of Drawings

FIG. 1 is a plan view of a catheter according to a first embodiment of the present invention.
FIG. 2 is a section view taken from a line A-A of FIG. 1.
FIG. 3 is a partial enlarged view illustrating a distal end portion of the catheter according to the first embodiment.
FIG. 4 is a partial section view taken from a line B-B of FIG. 3.
FIG. 5 is a section view of a catheter according to a second embodiment.
FIG. 6 is a partial enlarged view illustrating a distal end portion of the catheter according to the second embodiment.
FIG. 7 is a partial section view taken from a line C-C of FIG. 6.
FIG. 8 is a plan view of a catheter according to a third embodiment.
FIG. 9 is a partial enlarged view illustrating a distal end portion of the catheter according to the third embodiment.
FIG. 10 is a partial section view taken from a line D-D of FIG. 9.
FIG. 11 is an end face view taken from a line E-E of FIG. 9.
FIG. 12 is a partial enlarged view illustrating a distal end portion of a catheter according to a fourth embodiment.
FIG. 13 is a partial section view taken from a line F-F of FIG. 12.
FIG. 14 is a partial section view illustrating a distal end portion of a catheter according to a fifth embodiment.
FIG. 15 is a partial section view illustrating a distal end portion of a catheter according to a sixth embodiment.

### Description of Embodiments

In the following description, embodiments of the present invention will be described with reference to the enclosed drawings.

### (First embodiment)

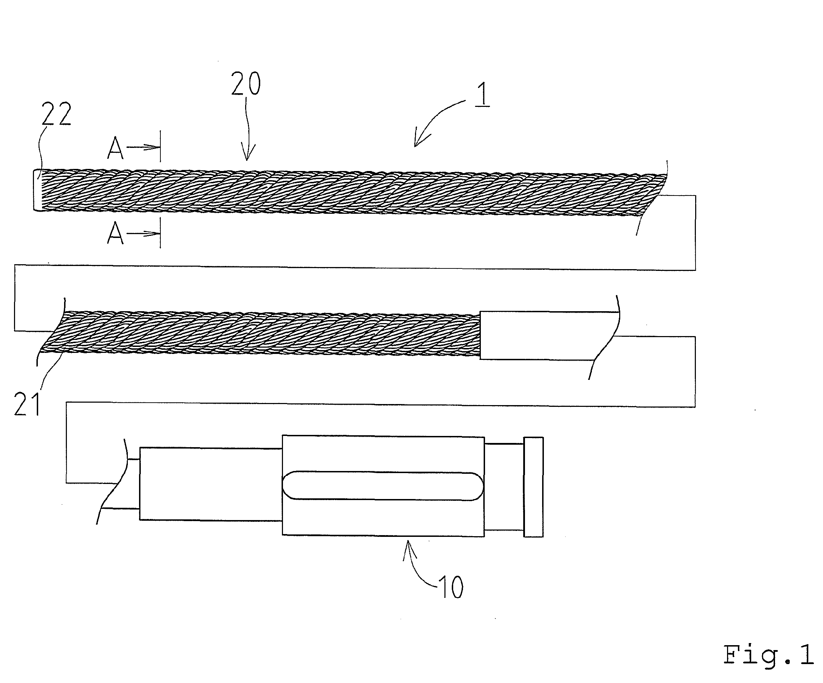

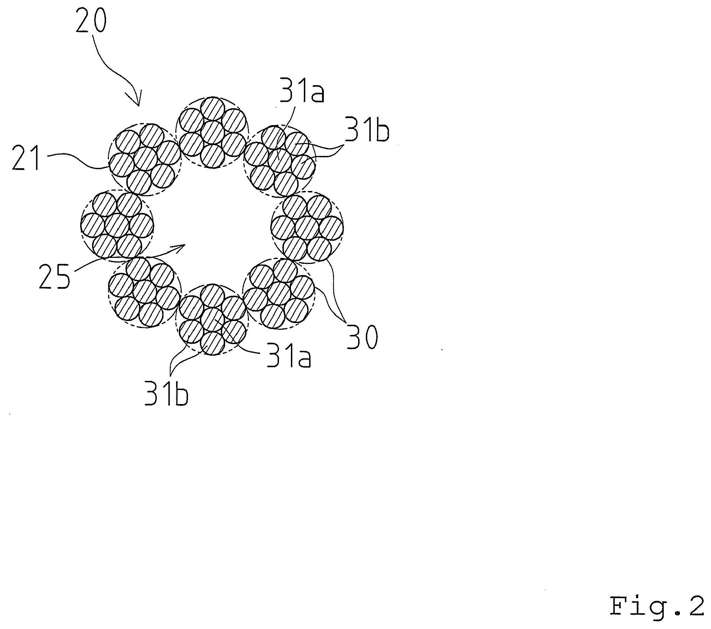

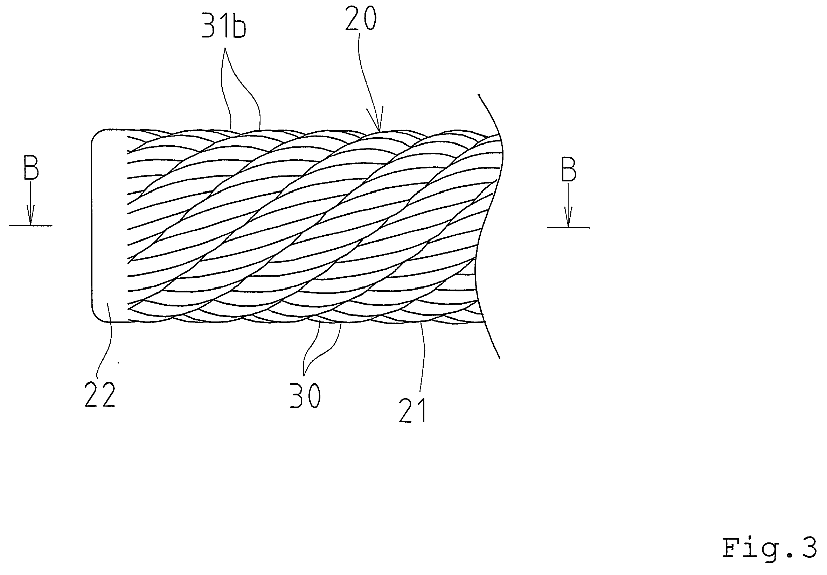

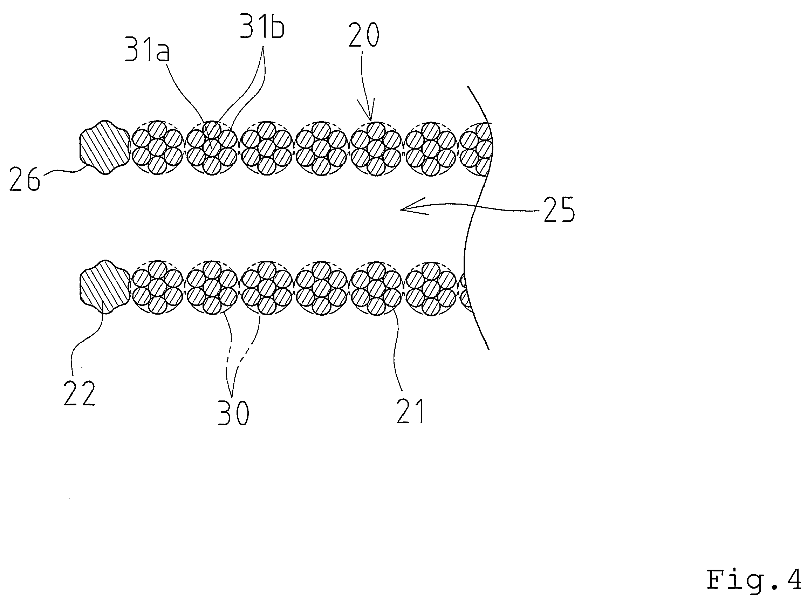

FIG. 1 is a plan view of a catheter according to the first embodiment of the present invention. FIG. 2 is a section view taken from a line A-A of FIG. 1. FIG. 3 is a partial enlarged view illustrating a distal end portion of the catheter according to the first embodiment. FIG. 4 is a partial section view taken from a line B-B of FIG. 3.

As illustrated in FIG. 1, a catheter 1 includes an operation part 10 operated by a technician and a coil body 20 connected to a distal end of the operation part 10.

Moreover, the coil body 20 includes a main body part 21 extended from the operation part 10 toward the distal end side, and a distal end portion 22 positioned on the distal end side of the main body part 21, and a hollow part 25 (see FIG. 2) is formed in the main body part 21 and the distal end portion 22.

Moreover, as illustrated in FIG. 2, the main body part 21 of the coil body 20 is tubular, in which eight bundles or pieces of stranded wires 30 are wound into a multi-line coil form. Note that the stranded wire 30 is formed by twisting six pieces of stainless steel second wire 31b as side wire around stainless steel first wire 31a as a core wire. Each second wire 31b and the first wire 31a constitutes a single wire. Accordingly, each of the eight stranded wires 30 is formed of seven single wires 31b, 31a (sixth single wires 31b and one single wire 31a). Thus, each stranded wire 30 may be considered as a bundle of seven single wires 31b, 31a.

Furthermore, as illustrated in FIG. 3 and FIG. 4, the distal end portion 22 of the coil body 20 is formed so that the ends of the stranded wires 30 positioned at the distal end portion 22 are fused and bonded by YAG welding, for example, to prevent the first wire 31a and the second wires 31b from coming undone, and includes a distal end opening portion 26 communicated to the hollow part 25.

In the catheter 1 of the present embodiment, the coil body 20 is formed by winding a plurality of pieces of stranded wires 30 into a helical coil structure. Thus, it is possible to improve torque transmissivity without impairing flexibility and improve insertion into a blocked portion of a blood vessel or the like.

Moreover, when the degree of curvature of the catheter 1 becomes large during use, it is possible to allow the catheter to easily restore its original form.

Moreover, as illustrated in FIG. 1 and FIG. 3, on the inner peripheral surface side and the outer peripheral surface of the coil body 20, a twisting direction of the second wires 31b forming the stranded wire 30 is substantially parallel to a long axis direction of the catheter. This improves insertion into a blocked portion of a blood vessel and the operability of a combination device such as a guide wire.

In the present embodiment, stainless steel is used as a material of the first wire 31a and the second wire 31b. However, other metal wires such as a superelastic alloy, such as an Ni-Ti alloy, a tungsten wire, or the like, may be used.

In the present embodiment, the number of single wires forming one stranded wire 30 is seven. However, the number of pieces of the first wire 31a and of the second wires 31b forming one stranded wire 30 is not especially limited as long as it is two or more. From the viewpoint of torque transmissivity, flexibility, and the like, the number is preferably three to ten.

Moreover, the coil body 20 may be formed by winding one stranded wire 30. However, when the coil body 20 is formed by winding a plurality of stranded wires 30, it is possible to further improve insertion into a blocked portion of a blood vessel and further improve the operability of a combination device such as a guide wire. When the catheter is curved, it is possible to allow the catheter to restore its original form more easily.

In the present embodiment, the distal end portion 22 is formed by fusing the first wire 31a and the second wires 31b forming the stranded wires 30. However, as another embodiment, the main body part 21 may be formed by a plurality of stranded wires 30, and the distal end portion 22 may be provided separately from the main body part 21. Also in such a case, the catheter 1 has excellent torque transmissivity and excellent flexibility, improving insertion into a blocked portion of a blood vessel and allowing the catheter to easily restore its original form when it is curved.

However, when the distal end portion 22 is formed by fusing the first wire 31a and the second wires 31b forming the stranded wires 30, it is possible to further improve insertion into a blocked portion of a blood vessel, the operability of a combination device such as a guide wire, and the shape restorability of the catheter.

Moreover, in the present embodiment, the distal end portion 22 is fused by welding the first wire 31a and the second wires 31b forming the stranded wires 30. However, the distal end portion 22 may be formed by a method other than welding. For example, the distal end portion 22 may be formed by brazing the first wire 31a and the second wires 31b.

### (Second embodiment)

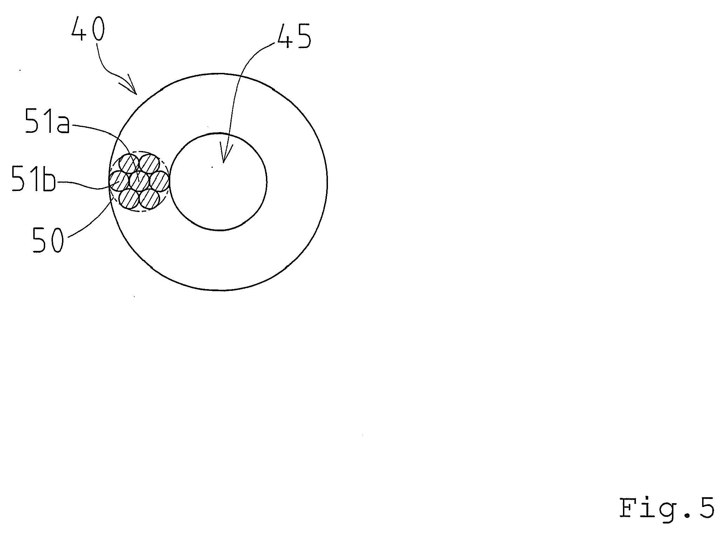

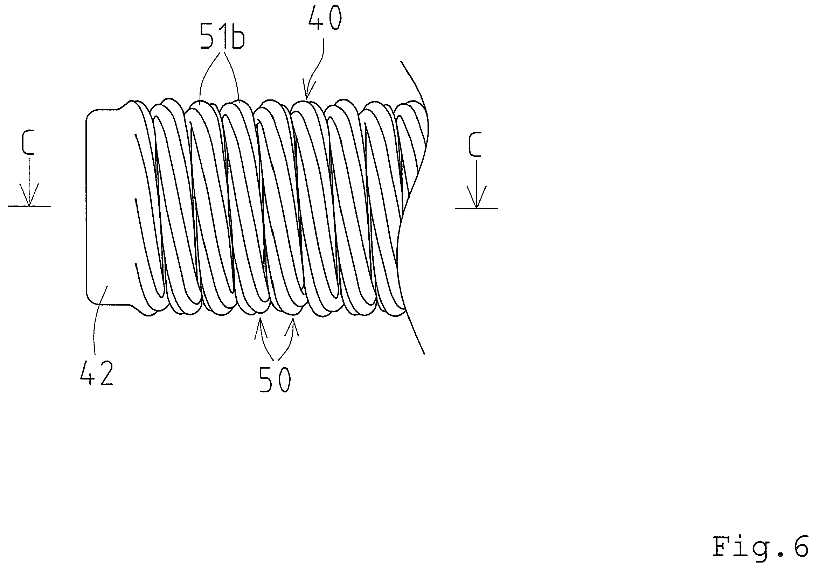

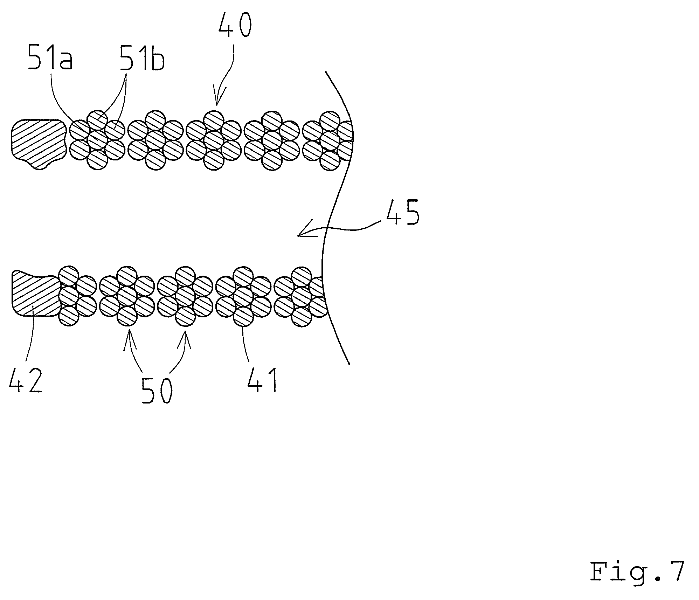

FIG. 5 is a section view of a catheter according to the second embodiment. FIG. 6 is a partial enlarged view illustrating a distal end portion of the catheter according to the second embodiment. FIG. 7 is a partial section view taken from a line C-C of FIG. 6.

As illustrated in FIG. 5, when the catheter according to the second embodiment is compared with the catheter 1 of the first embodiment, the number of stranded wires is different. That is, the main body part 21 of the coil body 20 of the first embodiment is wound by eight stranded wires 30 in a multi-line coil form (see FIG. 2), while a main body 41 of a coil body 40 of the second embodiment is wound by one stranded wire 30 into a single line coil form.

Note that the second embodiment is the same as the first embodiment in the aspects that a stranded wire 50 is formed by twisting six pieces of stainless steel second wire 51b as side wires around a stainless steel first wire 51a as a core wire; that the coil body 40 includes the main body part 41 extended from the operation part 10 toward the distal end side and a distal end portion 42 positioned on the distal end side of the main body part 41, and a hollow part 45 is formed in the main body part 41 and the distal end portion 42; and that the distal end portion 42 of the coil body 40 is formed so that the ends of stranded wire 50 positioned at the distal end portion 22 are fused and bonded by YAG welding, for example, to prevent the first wire 51a and the second wires 51b from coming undone.

The coil body 40 of the present embodiment is formed by winding one stranded wire 50 in a helical coil structure. Thus, it is possible to further improve flexibility compared to the catheter according to the first embodiment and to allow the catheter to easily restore its original position when it is curved.

However, as illustrated in FIG. 6, a twisting direction of the second wires 51b forming the stranded wire 50 is substantially vertical relative to a long axis direction of the catheter. Thus, the catheter according to the present embodiment is slightly inferior to the catheter according to the first embodiment in terms of insertion into a blocked portion of a blood vessel and operability of a combination device such as a guide wire.

### (Third embodiment)

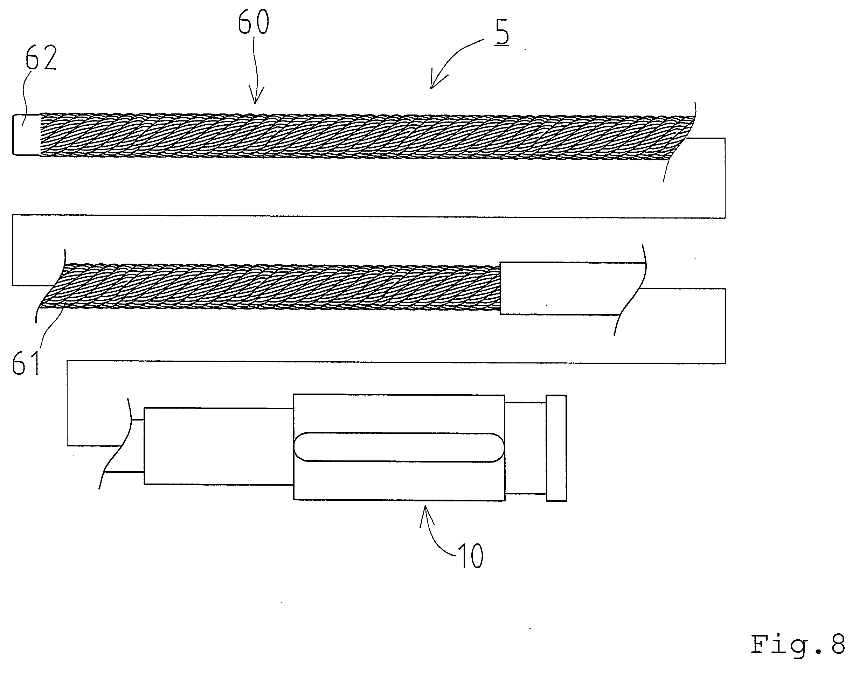

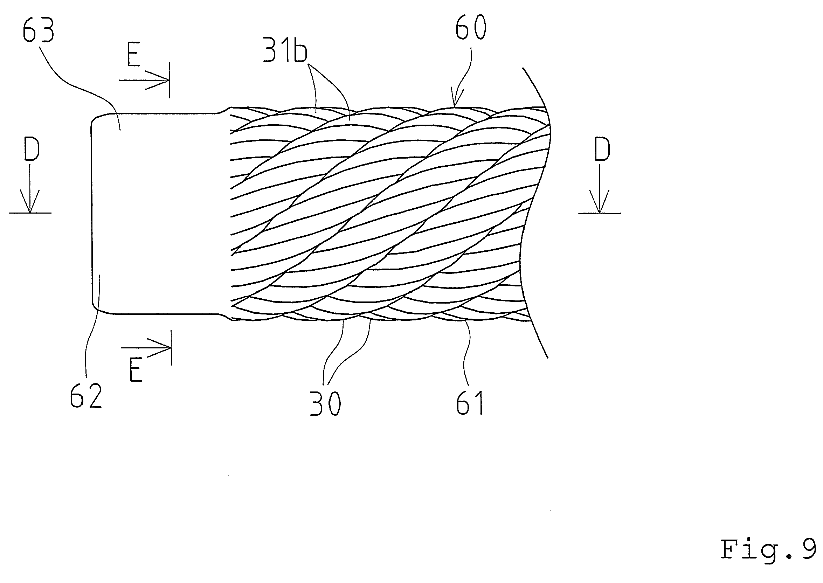

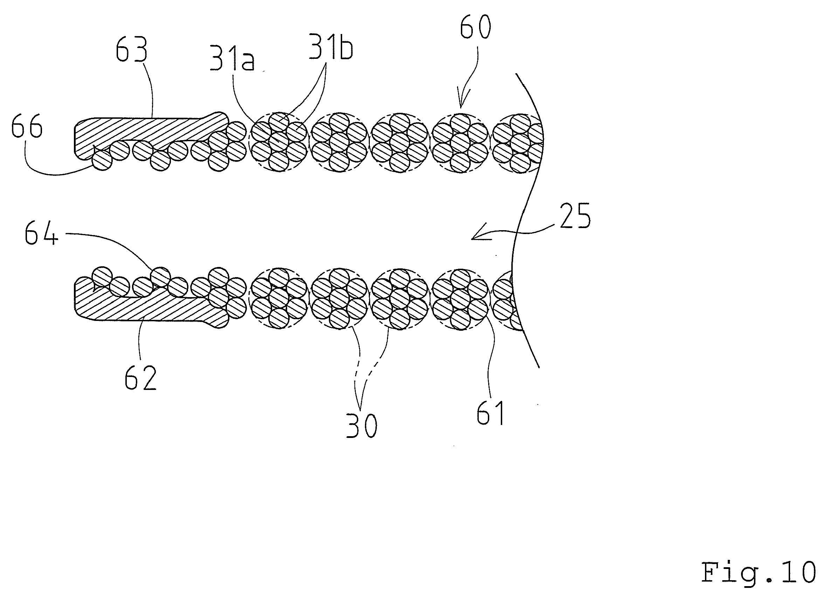

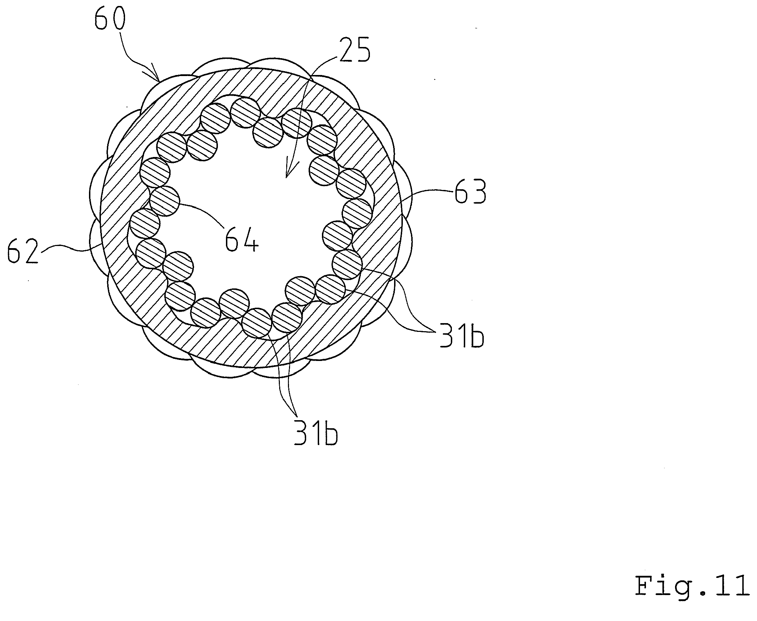

FIG. 8 is a plan view of a catheter according to the third embodiment. FIG. 9 is a partial enlarged view illustrating a distal end portion of the catheter according to the third embodiment. FIG. 10 is a partial section view taken from a line D-D of FIG. 9. FIG. 11 is an end face view taken from a line E-E of FIG. 9.

When the catheter 5 according to the third embodiment is compared with the catheter 1 of the first embodiment, the configuration of the distal end portion is different. That is, the distal end portion 22 of the coil body 20 of the first embodiment is formed by fusing the entirety of the first wire 31a and the second wires 31b forming the stranded wires 30. By contrast, regarding a distal end portion 62 of a coil body 60 of the third embodiment, the second wires 31b are not fused on an inner peripheral surface 64 at the distal end portion 62, and the form of the stranded wires 30 is kept, as illustrated in FIG. 10. That is, the inner peripheral surface 64 is uneven based on the outer form of the second wires 31b forming the stranded wires 30.

Note that the third embodiment is the same as the first embodiment in the aspect that the coil body 60 includes a main body part 61 extended from the operation part 10 toward the distal end side and the distal end portion 62 positioned on the distal end side of the main body part 61, and the hollow part 25 is formed in the main body part 61 and the distal end portion 62. In addition, the third embodiment is the same as the first embodiment also in the aspect that the main body part 61 of the coil body 60 is wound by eight stranded wires 30 in a multi-line coil form.

Meanwhile, as illustrated in FIG. 9, FIG. 10, and FIG. 11, on an outer peripheral surface 63 at the distal end portion 62, the first wire 31a and the second wires 31b forming the stranded wires 30 positioned at the distal end portion 62 are fused and bonded by YAG welding, for example, whereby the first wire 31a and the second wires 31b do not come undone, and the outer peripheral surface 63 at the distal end portion 62 is a smooth surface.

FIG. 11 illustrates a state where all first wires 31a and all second wires 31b positioned on the outer side are fused. However, a part of the first wire 31a and the second wire 31b may not be fused depending on a welding state. Even in such a case, it is sufficient if the outer peripheral surface 63 is flat.

By welding, it is possible to easily fuse only the first wire 31a and the second wires 31b corresponding to the outer peripheral surface 63 without fusing the first wire 31a and the second wires 31b corresponding to the inner peripheral surface 64.

A distal end opening portion 66 formed at the distal end of the distal end portion 62 is communicated to the hollow part 25 and, as illustrated in FIG. 10, the adjacent stranded wires 30 positioned at the distal end of the distal end portion 62 are fused so as not to come undone.

In the coil body 60 of the present embodiment, the outer peripheral surface 63 of the distal end portion 62 is a smooth surface. This reduces resistance when the catheter is inserted into a blocked portion of a blood vessel, for example, improving insertion into the blocked portion. By contrast, the inner peripheral surface 64 of the distal end portion 62 is uneven due to keeping the form of the stranded wires 30. This reduces a contact area between a combination device inserted into the hollow part 25 of the coil body 60, such as a guide wire, and the inner peripheral surface 64, suppressing sliding resistance and improving operability.

A twisting direction of the second wires 31b forming each stranded wire 30 is substantially parallel to a long axis direction of the catheter. Thus, it is possible to further improve the operability of a combination device such as a guide wire.

Moreover, the catheter 5 is configured integrally using the same member from the distal end portion 62 to the main body part 61 of the coil body 60, which prevents concentration of stress causing a break at a border area between the distal end portion 62 and the main body part 61.

Furthermore, the coil body 60 is formed by winding a plurality of stranded wires 30 into a multi-line coil form. Thus, the rigidity of the coil body 60 becomes larger, improving passing performance into a blocked portion or the like.

In the present embodiment, the smooth outer peripheral surface 63 and the inner peripheral surface 64 keeping the form of the stranded wires 30 are formed at the distal end portion 62 of the coil body 60. However, the main body part 61 may include a smooth outer peripheral surface and an inner peripheral surface keeping the form of stranded wire.

However, when the distal end portion 62 of the coil body 60 includes the smooth outer peripheral surface 63 and the inner peripheral surface 64 keeping the form of the stranded wire 30, it is not necessary to form the distal end portion using a separate part, which is advantageous.

In the present embodiment, the distal end portion 62 is formed by fusing the first wire 31a and the second wires 31b forming the stranded wires 30. However, as another embodiment, the main body part 61 may be formed by the stranded wires 30, and the distal end portion 62 may be provided separately from the main body part 61. Also in such a case, the catheter 1 has excellent torque transmissivity and excellent flexibility, improving insertion into a blocked portion of a blood vessel and allowing the catheter to easily restore its original form when it is curved.

However, when the distal end portion 62 is formed by fusing the first wire 31a and the second wires 31b forming the stranded wires 30, it is possible to further improve insertion into a blocked portion of a blood vessel, the operability of a combination device such as a guide wire, and the shape restorability of the catheter.

Moreover, in the present embodiment, the distal end portion 62 is fused by welding the first wire 31a and the second wires 31b forming the stranded wires 30. However, the distal end portion 62 may be formed by a method other than welding. For example, the distal end portion 62 may be formed by brazing the first wire 31a and the second wires 31b.

### (Fourth embodiment)

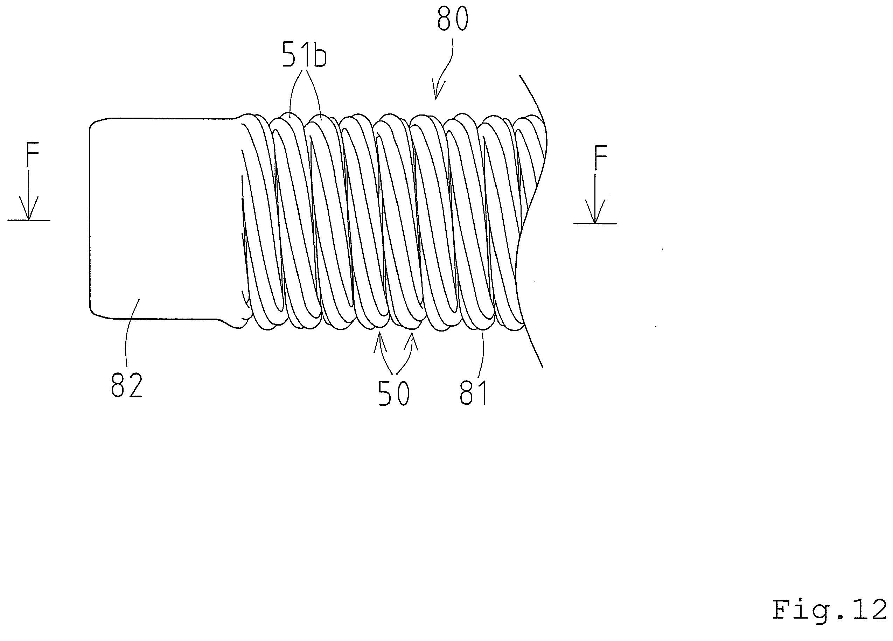

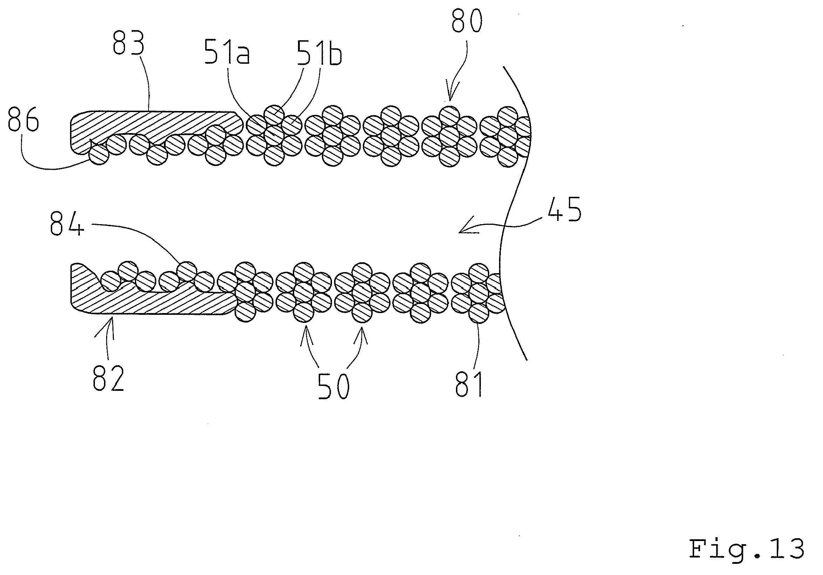

FIG. 12 is a partial enlarged view illustrating a distal end portion of a catheter according to the fourth embodiment. FIG. 13 is a partial section view taken from a line F-F of FIG. 12.

When the catheter according to the fourth embodiment is compared with the catheter 5 of the third embodiment, the number of stranded wires is different. That is, the main body part 61 of the coil body 60 of the third embodiment is wound by eight stranded wires 30 in a multi coil form, while a main body 81 of a coil body 80 of the fourth embodiment is wound by one stranded wire 50 in a single coil form.

Note that the fourth embodiment is the same as the third embodiment in the aspects that the stranded wire 50 is formed by twisting six pieces of stainless steel second wires 51b as side wires around a stainless steel first wire 51a as a core wire; that the coil body 80 includes the main body part 81 extended from the operation part 10 toward the distal end side and a distal end portion 82 positioned on the distal end side of the main body part 81, and the hollow part 45 is formed in the main body part 81 and the distal end portion 82; and that the distal end portion 82 of the coil body 80 is formed so that the ends of the stranded wire 50 positioned at the distal end portion 82 are fused and bonded by YAG welding, for example, to prevent the first wire 51a and the second wires 51b from coming undone.

Meanwhile, as illustrated in FIG. 13, on an outer peripheral surface 83 of the distal end portion 82, the first wire 51a and the second wires 51b forming the stranded wire 50 positioned at the distal end portion 82 are fused and bonded by YAG welding, for example, whereby the first wire 51a and the second wires 51b do not come undone, and the outer peripheral surface 83 at the distal end portion 82 is a smooth surface.

By welding, it is possible to easily fuse only the first wire 51a and the second wires 51b corresponding to the outer peripheral surface 83 without fusing the first wire 51a and the second wires 51b corresponding to the inner peripheral surface 84.

A distal end opening portion 86 formed at the distal end of the distal end portion 82 is communicated to the hollow part 45, and the adjacent stranded wire 50 positioned at the distal end of the distal end portion 82 is fused so as not to come undone.

Also in the coil body 80 of the present embodiment, the outer peripheral surface 83 of the distal end portion 82 is a smooth surface. This reduces resistance when the catheter is inserted into a blocked portion of a blood vessel or the like, improving insertion into a blocked portion. By contrast, the inner peripheral surface 84 of the distal end portion 82 is uneven due to keeping the form of the stranded wire 50. This reduces a contact area between a combination device inserted into the hollow part 45 of the coil body 80, such as a guide wire, and the inner peripheral surface 84, suppressing sliding resistance and improving operability.

Moreover, the catheter 1 is formed integrally using the same member from the distal end portion 82 to the main body part 81 of the coil body 80, which prevents concentration of stress causing a break at a border area between the distal end portion 82 and the main body part 81.

In the present embodiment, the smooth outer peripheral surface 83 and the inner peripheral surface 84 keeping the form of the stranded wire 50 are formed at the distal end portion 82 of the coil body 80. However, the main body part 81 may include a smooth outer peripheral surface and an inner peripheral surface keeping the form of stranded wire. However, when the distal end portion 82 of the coil body 80 includes the smooth outer peripheral surface 83 and the inner peripheral surface 84 keeping the form of the stranded wire 50, it is not necessary to form the distal end portion using a separate part, which is advantageous.

### (Fifth embodiment)

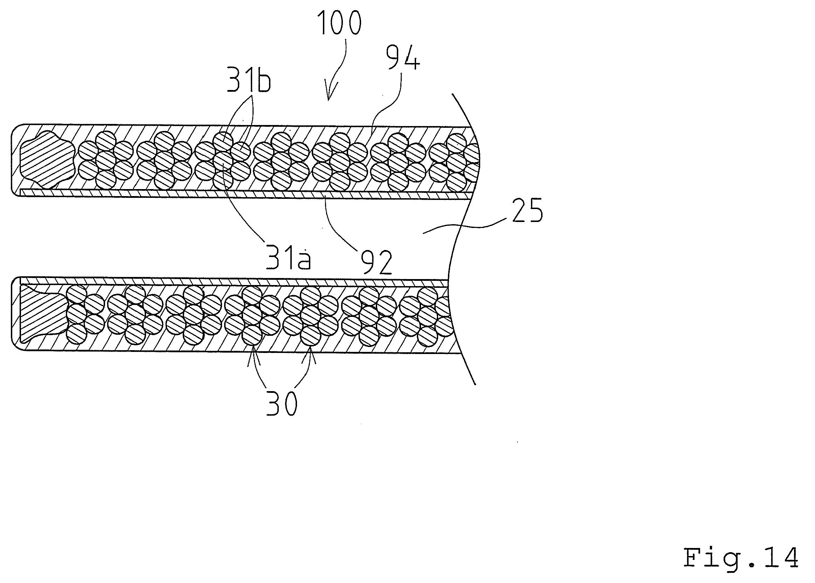

FIG. 14 is a partial section view illustrating a distal end portion of a catheter according to the fifth embodiment.

The catheter according to the fifth embodiment is formed by covering the outer periphery of the catheter according to the first embodiment with resin. To be more specific, an inner layer 92 is formed along the hollow part 25 in a coil body 100, and an outer layer 94 is formed on the outer side of the inner layer 92 so as to cover the coil body 100. As shown in Fig. 14, the stranded wires 30 are enclosed within and covered by the outer layer 94.

The catheter according to the fifth embodiment has excellent torque transmissivity and excellent flexibility, further improving insertion into a blocked portion of a blood vessel and further improving the operability of a combination device such as a guide wire.

The catheter according to the present embodiment is formed by covering the outer periphery of the catheter according to the first embodiment with resin. However, the outer periphery of the catheter according to the second embodiment may be covered with resin.

Alternatively, the outer periphery of a catheter, in which the distal end portion of the coil body 20 of the first embodiment or the distal end portion of the coil body 40 of the second embodiment is provided separately from the main body part 21 or the main body part 41, may be covered with resin.

### (Sixth embodiment)

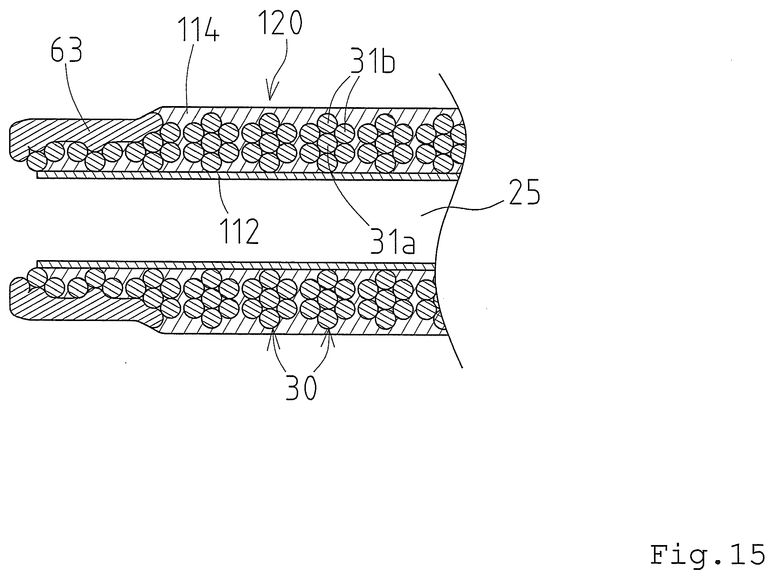

FIG. 15 is a partial section view illustrating a distal end portion of a catheter according to the sixth embodiment.

The catheter according to the six embodiment is formed by covering a part of the outer periphery of the catheter according to the third embodiment with resin. To be more specific, an inner layer 112 is formed along the hollow part 45 in a coil body 120, and an outer layer 114 is formed on the outer side of the inner layer 112 so as to cover the coil body 120 in a place other than the outer peripheral surface 63.

The catheter according to the sixth embodiment has excellent torque transmissivity and excellent flexibility, further improving insertion into a blocked portion of a blood vessel and further improving the operability of a combination device such as a guide wire.

The catheter according to the present embodiment is formed by covering the outer periphery of the catheter according to the third embodiment with resin. However, the outer periphery of the catheter according to the fourth embodiment may be covered with resin.

Alternatively, the outer periphery of a catheter, in which the distal end portion of the coil body 60 of the third embodiment or the distal end portion of the coil body 80 of the fourth embodiment is provided separately from the main body part 61 or the main body part 81, may be covered with resin.

### Reference Signs List

1, 5 catheter
10 operation part
20, 40, 60, 80, 100, 120 coil body
21, 41, 61, 81 main body part
22, 42, 62, 82 distal end portion
63, 83 outer peripheral surface
64, 84 inner peripheral surface
25, 45 hollow part
26, 66, 86 distal end opening portion
30, 50 stranded wire
31a, 51a first wire (single wire)
31b, 51b second wire (single wire)
92, 112 inner layer
94, 114 outer layer

## Claims

1. A catheter (1, 5), comprising:
a coil body (20, 40, 60, 80, 100, 120) formed of at least one stranded wire (30, 50) which is formed of a plurality of single wires (31a, 31b, 51a, 51b) and is wound into a hollow helical coil structure.

2. The catheter (1, 5) according to claim 1, wherein the coil body (20, 60, 100, 120) is formed of a plurality of stranded wires (30) which are wound into the hollow helical coil structure, wherein each of the plurality of stranded wires (30) is formed of a plurality of single wires (31a, 31b, 51a, 51b).

3. The catheter (1) according to claim 1 or 2, wherein a distal end portion of the coil body (20, 40, 100) is formed by fusing the single wires (31a, 31b, 51a, 51b) of the at least one stranded wire (30, 50).

4. The catheter (5) according to claim 1 or 2, wherein a distal end portion of the coil body (60, 80, 120) includes an outer surface (63, 83) formed by fusing the single wires (31a, 31b, 51a, 51b) of the at least one stranded wire, and an inner surface (64, 84) keeping a form of the stranded wire.

5. The catheter (1) according to any one of claims 1 to 4, further comprising:
an inner layer (92) formed inside of the coil body (100); and
an outer layer (94) formed outside of the inner layer (92) so as to cover the coil body (100).

6. The catheter (5) according to claim 3 or 4, further comprising:
an inner layer (112) formed inside of the coil body (120); and
an outer layer (114) formed outside of the inner layer (112) so as to cover the coil body (120) in an area other than the distal end portion of the coil body (120).
